# EUROPÄISCHE PATENTSCHRIFT

(11) **EP 3 184 569 B1**
(45) Veröffentlichungstag und Bekanntmachung des Hinweises auf die Patenterteilung: **26.09.2018**
(21) Anmeldenummer: 15202260.4
(22) Anmeldetag: 23.12.2015
(51) Int. Cl.: C08G 59/68, C07F 5/06, C08F 4/52, C08G 65/10

(54) **HÄRTUNGSINITIATOREN FÜR DIE KATIONISCHE POLYMERISATION**
CURING INITIATORS FOR CATIONIC POLYMERIZATION
INITIATEURS DE DURCISSEMENT POUR LA POLYMERISATION CATIONIQUE

(43) Veröffentlichungstag der Anmeldung: 28.06.2017
(73) Patentinhaber: Fraunhofer-Gesellschaft zur Förderung der angewandten Forschung e.V., 80686 München (DE)
(72) Erfinder: Hartwig, Andreas, 27721 Ritterhude (DE); Redetzky, Maurice, 28195 Bremen (DE); Krieger, Antonina, 27283 Verden (DE)
(74) Vertreter: Glawe, Delfs, Moll

(56) Entgegenhaltungen:
- EP-B1- 2 223 948
- WO-A1-98/40421
- WO-A1-99/12938
- WO-A1-2015/040480
- ANDREAS BÖSMANN ET AL: "Activation, Tuning, and Immobilization of Homogeneous Catalysts in an Ionic Liquid/Compressed CO2 Continuous-Flow System", ANGEWANDTE CHEMIE INTERNATIONAL EDITION, Bd. 40, Nr. 14, 16. Juli 2001 (2001-07-16) , Seiten 2697-2699, XP055261874, DE ISSN: 1433-7851, DOI: 10.1002/1521-3773(20010716)40:14<2697::AID -ANIE2697>3.0.CO;2-W

## Beschreibung

Die Erfindung betrifft Formulierungen, die eine als Härtungsinitiator für die kationische Polymerisation geeignete Zusammensetzung sowie kationisch polymerisierbare Monomere, Oligomere und/oder Präpolymere enthalten und deren Verwendung.

Die kationische Polymerisation von Epoxidharzen und Vinylethern wird vielfältig bei Klebstoffen, Vergussmassen, Lacken, Matrixharzen für Verbundwerkstoffe oder Imprägnierharzen eingesetzt. Hierbei werden üblicherweise latente Initiatoren zugesetzt, welche dann thermisch oder photochemisch aktiviert werden.

Bei diesen Initiatoren handelt es sich typischerweise um organische Salze, welches ein Iodonium-, Sulfonium-, Ammonium- oder Phosphoniumkation beinhalten und PF₆⁻ oder SbF₆⁻ als Anion. In der Grundlagenliteratur beschriebene Anionen wie BF₄⁻ oder AsF₆⁻ konnten sich technisch nicht durchsetzen.

In der Grundlagen- und Patentliteratur sind zahlreiche Initiatoren nach diesem Aufbauprinzip beschrieben. Es kommt aber immer wieder vor, dass die verwendeten Anionen Fluorid abspalten, welches zu Korrosion von Bauteilen führen kann und zudem toxisch ist. Bei dem besonders aktiven SbF₆⁻ wird das enthaltene Antimon unter toxikologischen und Umweltgesichtspunkten kritisch betrachtet.
Es hat daher in der Vergangenheit nicht an Versuchen gefehlt alternative Anionen für derartige Katalysatoren zu finden. So wird in der US 5,693,688 [B(C₆F₅)₄]⁻ vorgeschlagen und in der EP 2 223 948 B1 entsprechende Anionen, bei denen vereinzelte Fluoratome dieses Anions gegen andere Substituenten ausgetauscht wurden. Durch die Bor-Kohlenstoff-Bindung sind derartige Anionen nur sehr schwer herzustellen, daher teuer und werden daher nur sehr vereinzelt in der Technik angewandt.
WO 2015/040480 A1 offenbart ein Verfahren zur Synthese von gemischten Borhydriden der allgemeinen Former MₓM'_{y}(BH₄)₂. Bösmann et al., Angewandte Chemie International Edition, Bd. 40, Nr. 14, 2001, S. 2697-2699, offenbart die Aktivierung, die Abstimmung und die Immobilisation von homogenen Katalysatoren in ionischen Flüssigkeiten.

Die Verwendung von Aryliodonium- und/oder Arylsulfoniumsalzen des Tetrakis(perfluor-t-butyloxy)aluminat-Anions als Initiatoren bei der Polymerisation kationisch polymerisierbarer Monomere wird in der EP 3 300 504 A1 (WO 2017/035552 A1) beschrieben, wobei das Dokument Stand der Technik nach Art. 54(3) EPÜ darstellt.

Es besteht daher ein Bedarf nach effektiven latenten Initiatoren für die kationische Polymerisation insbesondere von Epoxidharzen und Vinylethern mit alternativen Anionen.
Aufgabe der vorliegenden Erfindung ist es daher, mit Zusammensetzungen mit alternativen Anionen für latente Initiatoren hergestellte härtbare Formulierungen auf der Basis von Epoxidharzen und Vinylethern bereit zu stellen.
Gelöst wird diese Aufgabe durch eine Formulierung nach Anspruch 1. Vorteilhafte Ausgestaltungen der Erfindung sind in den Unteransprüchen angegeben.

Der Begriff Zusammensetzung umfasst im Rahmen der Erfindung sowohl Verbindungen (organische Salze), die die anspruchsgemäßen Kationen und Anionen enthalten als auch Zusammensetzungen wie beispielsweise Lösungen, die die anspruchsgemäßen Kationen und Anionen enthalten. Wenn im Rahmen der Erfindung Mengenanteile der Zusammensetzung definiert werden bei der Verwendung bzw. bei der Definition erfindungsgemäßer Formulierungen, beziehen sich diese Mengenanteile immer auf die reine Verbindung aus den definierten Kationen und Anionen.

Überraschend hat sich gezeigt, dass Verbindungen, welche ein organisches Sulfonium-, Iodonium-, Ammonium- oder Phosphoniumion als Kation und ein Anion [E(ZR1)ₙ(ZR2)ₘ] enthalten, sich hervorragend als latente Initiatoren für die kationische Polymerisation insbesondere von Epoxidharzen und Vinylethern eignen.

Hierbei ist E Aluminium oder Bor und Z Sauerstoff oder Schwefel.

Erfindungsgemäß sind die organischen Reste R1, R2 demnach über Sauerstoff oder Schwefel an das Aluminium oder Bor gebunden. Hierbei können die Reste R1, R2 gleich oder verschieden sein, mindestens zwei Reste R2 der vier Reste beinhalten aber an Kohlenstoff gebundenes Fluor.

Um eine für Anwendungen hinreichende Aktivität der mit den Anionen hergestellten erfindungsgemäßen Initiatoren zu erzielen, müssen bei den fluorhaltigen Resten R2 mindestens vier, vorzugsweise wenigstens die Hälfte der zur Absättigung der Kohlenstoffatome benötigten Wasserstoffe durch Fluor ersetzt sein. Die zur Absättigung der Kohlenstoffatome benötigten Wasserstoffe sind diejenigen H-Atome, die unmittelbar an Kohlenstoff gebunden sind. Bevorzugt sind insbesondere H-Atome an nahe an E angeordneten C-Atomen durch F ersetzt. Für die Aktivität der Initiatoren ist es besonders wichtig, dass die sich nahe am der Sauerstoff- oder Schwefelbrücke zum Aluminium oder Bor befindlichen Kohlenstoffe mit Fluor substituiert sind. Hingegen haben Fluorsubstituenten an weiter entfernten Kohlenstoffatomen nur noch eine kleine Wirkung auf die Aktivität der als Initiator fungierenden Zusammensetzung. Wenn der Substituent R2 mehr als 7 Kohlenstoffatome aufweist, so bezieht sich die mindestens 50%ige Substitution der zur Absättigung der Kohlenstoffe notwendigen Wasserstoffe auf die 7 Kohlenstoffatome die der Sauerstoff- oder Schwefelbrücke zum Aluminium oder Bor am nächsten sind.

Entsprechende Anionen sind an sich im Stand der Technik bekannt und werden insbesondere in der WO 99/12938 A1 beschrieben. Hier werden sie in Kombination mit Lithium als Kation für die Anwendung in Batterien vorgeschlagen und in Kombination mit katalytisch wirkenden Metallkationen um deren katalytische Aktivität zu verbessern.

Die organischen Reste R1 beinhalten 1 bis 30 Kohlenstoffatome, können linear oder verzweigt sein, cyclisch sein oder Cyclen beinhalten, aromatisch sein oder aromatische Ringe beinhalten, Mehrfachbindungen enthalten und auch die in der organischen Chemie üblichen Substituenten enthalten. Beispielhaft seien hier Chlor, Brom, Ether, Ester, Keton, Aldehyd, Nitro und Nitroso genannt.

Die organischen Reste R2 beinhalten 2 bis 30 Kohlenstoffatome, bevorzugt 3 bis 15 Kohlenstoffatome, können linear oder verzweigt sein, cyclisch sein oder Cyclen beinhalten, aromatisch sein oder aromatische Ringe beinhalten, Mehrfachbindungen enthalten und auch die in der organischen Chemie üblichen Substituenten enthalten. Beispielhaft seien hier Chlor, Brom, Ether, Ester, Keton, Aldehyd, Nitro und Nitroso genannt. Bei den Resten R2 ist vorzugsweise mindestens die Hälfte der zur Absättigung der Kohlenstoffatome notwendigen Wasserstoffe gegen Fluor ausgetauscht. Bevorzugt ist der gesamte zur Absättigung der Kohlenstoffatome notwendige Wasserstoff durch Fluor ersetzt. Für die Aktivität der Initiatoren ist es besonders wichtig, dass die sich nahe am der Sauerstoff- oder Schwefelbrücke zum Aluminium oder Bor befindlichen Kohlenstoffe mit Fluor substituiert sind. Hingegen haben Fluorsubstituenten an weiter entfernten Kohlenstoffatomen nur noch eine kleine Wirkung auf die Aktivität der als Initiator fungierenden Zusammensetzung. Wenn der Substituent R2 mehr als 7 Kohlenstoffatome aufweist, so bezieht sich die mindestens 50%ige Substitution der zur Absättigung der Kohlenstoffe notwendigen Wasserstoffe auf die 7 Kohlenstoffatome die der Sauerstoff- oder Schwefelbrücke zum Aluminium oder Bor am nächsten sind.

Mindestens die Hälfte der Reste R sind R2 und n+m=4. Bevorzugt sind die organischen Reste R gleich und sind R2 (d.h. m=4).

Bevorzugte Anionen sind Aluminatanionen der allgemeinen Formel [Al(OR2)₄]⁻ und Boratanionen der allgemeinen Formel [B(OR2)₄]⁻ mit den bereits definierten organischen Resten R2, wobei mindestens die Hälfte der zur Absättigung der Kohlenstoffatome notwendigen Wasserstoffe durch Fluor ersetzt sind und besonders bevorzugt sind alle zur Absättigung der Kohlenstoffatome notwendigen Wasserstoffe durch Fluor ersetzt sind.

Besonders bevorzugt sind die folgenden Reste R2, wobei - die Bindungsstelle zum Sauerstoff oder Schwefel und Ph ein Benzolring
ist: -C(CF₃)₃, -CH(CF₃)₂, -C(CH₃)(CF₃)₂, -CPh(CF₃)₂, -CPh₂(CF₃),-CPhCH₃(CF₃)₂, -CPhCF₃(CF₃)₂, -PhF₅, -PhF₄, -PhClF₄.

Bei -CPhCH₃(CF₃)₂, -CPhCF₃(CF₃)₂, -PhF₄ und -PhClF₄ können die Substituenten beliebig am Benzolring angeordnet sein, bevorzugt werden aber folgende Strukturen: 4-Methyltetrafluorbenzoxy, 4-Trifluormethylbenzoxy, 2,3,5,6-Tetrafluorbenzoxy, 4-Chlortetrafluorbenzoxy, wobei das "oxy" bereits den Sauerstoff, über den die Anbindung an das Aluminium oder Bor erfolgt, beinhaltet.

Die Reste R1 und R2 können auch miteinander chemisch verknüpft sein und somit einen bidentalen Rest R3 bilden, der zwei Bindungsstellen über jeweils ein Sauerstoffatom zum Bor oder Aluminium aufweist. Bei einem solchen bidentalen Rest R3 bezieht sich die anspruchsgemäße Anzahl der C-Atome auf diesen gesamten Rest R3. Ein solcher bidentaler Rest R3 weist somit erfindungsgemäß 2 bis 30 C-Atome, bevorzugt 3 bis 15 C-Atome auf. Die Mindestzahl von 2 ist erforderlich, um einen bidentalen Rest bilden zu können. Diese Anionen haben demnach die folgenden allgemeinen Formeln:

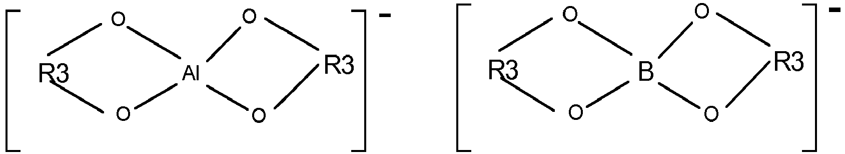

Beispiele für bevorzugte R3 sind 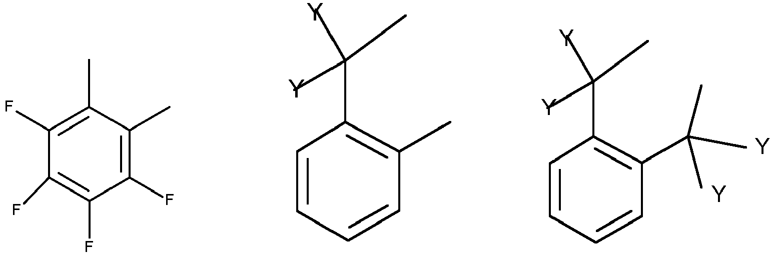 wobei die freien Bindungsstellen zu den die Bindung zum Aluminium oder Bor bildenden Sauerstoffen gehören. Y ist -CF₃ oder - F und die weiteren mit Wasserstoff substituierten Bindungsstellen an den Benzolringen können weitere Substituenten tragen, bevorzugt -CF₃ oder -F.

Bei dem Kation des Initiators handelt es sich um ein organisch substituiertes Iodonium-, Sulfonium-, Phosphonium oder Ammoniumkation. Diese haben die allgemeinen Formeln (Rx)₂I⁺, (Rx)₃S⁺, (Rx)₄P⁺, oder (Rx)₄N⁺ mit Rx ausgewählt aus der Gruppe bestehend aus R4, R5, R6 und R7.

Bei den Resten Rx handelt es sich um die üblichen organischen Substituenten. Die organischen Reste Rx beinhalten 1 bis 30 Kohlenstoffatome, bevorzugt 3 bis 15 Kohlenstoffatome, können linear oder verzweigt sein, cyclisch sein oder Cyclen beinhalten, aromatisch sein oder aromatische Ringe beinhalten oder Heterocyclen sein, Mehrfachbindungen enthalten und auch die in der organischen Chemie üblichen Substituenten enthalten. Beispielhaft seien hier Chlor, Fluor, Brom, Ether, Ester, Keton, Hydroxyl, Carbonsäure, Aldehyd, Nitro und Nitroso genannt.

Die Reste Rx können gleich oder verschieden sein, wenigstens einer der Rx muss aber ein aromatischer Ring sein oder einen aromatischen Ring beinhalten. Die aromatischen Ringe sind bevorzugt mit organischen Resten substituiert, welche 1 bis 15 Kohlenstoffatome beinhalten. Besonders bevorzugt sind Pyridiniumderivate und die nachfolgend aufgeführten Kationen:

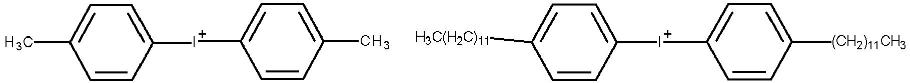

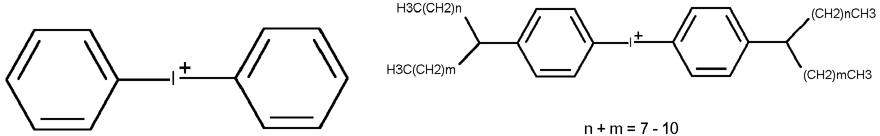

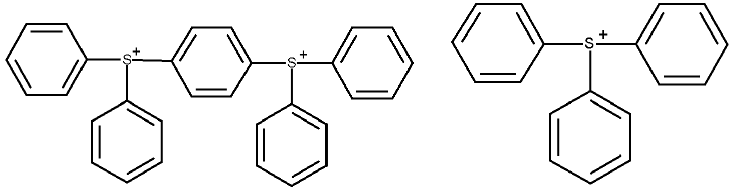

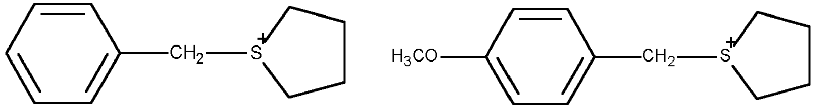

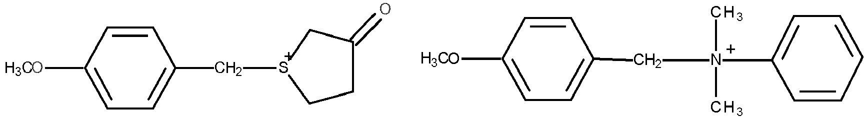

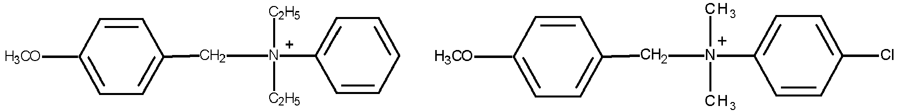

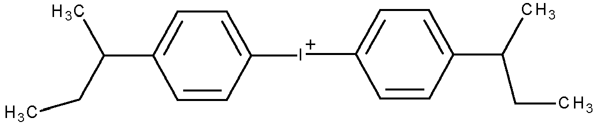

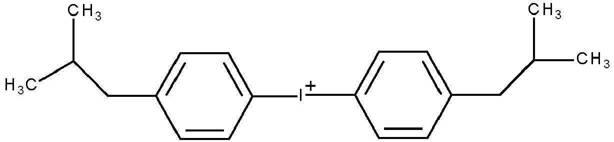

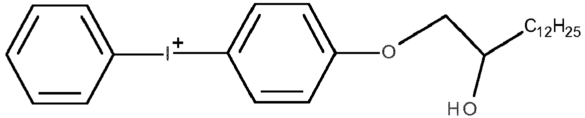

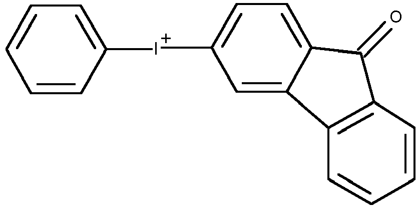

Besonders bevorzugte Kationen sind:

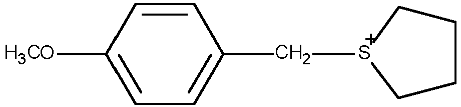

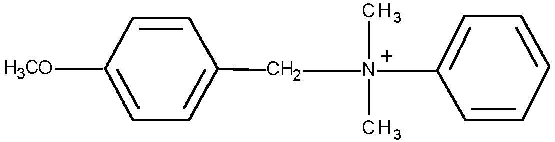

Es ist jede beliebige Kombination aus den dargelegten Anionen und Kationen möglich.

Die Herstellung der Initiatoren erfolgt durch eine Metathesereaktion. Hierbei wird ein das Anion und ein das Kation beinhaltendes Salz in bevorzugt äquimolaren Mengen jeweils in einem Lösemittel gelöst und die Lösungen vereinigt, wobei der Initiator ausfällt und das als Nebenprodukt entstehende Salz in Lösung bleibt. Der Niederschlag wird abfiltriert, mit dem Lösemittel gewaschen und getrocknet. Hierfür wird das Kation des das Aluminat- oder Boratanion enthaltenden Salzes und das Anion des als Ausgangsstoff dienenden Oniumsalzes so gewählt, dass das daraus gebildete Salz in dem Lösemittel gut löslich ist. In dieser Variante ist Wasser ein geeignetes Lösemittel und typische Anionen sind Halogenidionen und typische Kationen Alkalimetallionen.

Eine andere Form der Metathesereaktion ist die, dass die Reaktion in einem Lösemittel durchgeführt wird in dem der Initiator bestehend aus Oniumion und Aluminat- oder Boratanion gut löslich ist und das als Nebenprodukt entstehende Salz unlöslich. Hierfür sind organische Lösemittel geeignet und nach erfolgter Reaktion wird der Niederschlag aus dem Salz abfiltriert und der Initiator durch Verdampfen des Lösemittels gewonnen.

Ein weiterer Gegenstand der Beschreibung ist die Verwendung einer anspruchgemäßen Zusammensetzung als Polymerisationsinitiator für eine kationische Polymerisation. Bevorzugt wird die Zusammensetzung der zu polymerisierenden Masse in einem Anteil von 0,05 bis 10 Gew.-%, weiter vorzugsweise 0,2 bis 5 Gew.-% zugesetzt.

Die Polymerisation wird bevorzugt durch thermische Anregung oder Anregung mit Licht initiiert, wobei Licht bevorzugt im Wellenlängenbereich von 250 bis 600 nm, weiter vorzugsweise 300 bis 500 nm eingesetzt wird.

Denkbar ist die Verwendung für eine kationische Polymerisation von Epoxidharzen und/oder Vinylethern.

Erfindungsgemäß werden Formulierungen bereitgestellt, die kationisch polymerisierbare Monomere, Oligomere und/oder Präpolymere (bevorzugt Epoxidharze oder Vinylether) und einen der anspruchsgemäßen Initiatoren bzw. eine anspruchsgemäße Zusammensetzung beinhalten. Die Zusammensetzungen bzw. Initiatoren werden bevorzugt in Anteilen von 0,05 bis 10 Gew.-% zugesetzt, weiter bevorzugt 0,2 bis 5 Gew.-%.

Es resultiert eine latente Reaktionsmischung, welche durch einen externen Stimulus zum Aushärten gebracht wird. Der Stimulus für die Initiierung der Härtungsreaktion ist die Anregung mit Licht, bevorzugt im Wellenlängenbereich von 250 bis 600 nm und besonders bevorzugt zwischen 300 und 500 nm, oder eine thermische Anregung durch Erwärmen der Reaktionsmischung.

Als Epoxidharze kommen alle im Stand der Technik üblichen Epoxidharze in Frage, bevorzugt Glycidylether des Bisphenol A und Bisphenol F bzw. deren Oligomeren, Glycidylether von Phenolharzen, epoxidierte ungesättigte Kohlenwasserstoffe, beispielweise epoxidierte Pflanzenöle, hier vor allem epoxidiertes Leinöl, epoxidiertes Sojaöl oder epoxidiertes Rizinusöl, epoxidiertes Limonen und vor allem cycloaliphatische Epoxidharze. Letztere sind dadurch gekennzeichnet, dass die Epoxidgruppe direkt an einen aliphatischen 5- oder 6-Ring gebunden ist. 3,4-Epoxycyclohexyl-3',4'-epoxycyclohexane carboxylat und Bis-(3,4-epoxycyclohexylmethyl)-adipat sind besonders bevorzugte cycloaliphatische Epoxidharze.

Beispielhaft geeignete Vinylether sind Cyclohexylvinylether, Butandiolvinylether, Triethylenglycoldivinylether, VEctomer® 1222 Vinylether, VEctomer® 2032 Vinylether, VEctomer® 1312 Vinylether, VEctomer® 2010 Vinylether oder VEctomer® 2020 Vinylether.

Die erfindungsgemäßen Formulierungen aus Epoxidharzen oder Vinylethern und den anspruchsgemäßen als Initiatoren fungierenden Zusammensetzungen können weitere Bestandteile enthalten.

Es werden mehrwertige Alkohole zugegeben, sogenannte Polyole. Diese beinhalten 2 bis 5 OH-Gruppen je Molekül, bevorzugt 2 oder 3 OH-Gruppen und weisen Molmassen von 50 bis 20.000, bevorzugt 200 bis 6000, auf. Die Polyole sind in einem Anteil von 5 bis 80 Gew.-% der Formulierung, vorzugsweise 10 bis 50 Gew.-% der Formulierung enthalten.

Als chemische Basis der Polyole zur Einstellung der mechanischen Eigenschaften der Formulierungen und zur Modifizierung der Reaktivität eignen sich besonders Polypropylenoxid, Polyethylenoxid, Polycaprolacton, Polyhexandioladipat und weitere im Stand der Technik bekannte Polyester.

Bevorzugte Formulierungsbestandteile sind auch Oxetane, durch deren Zusatz insbesondere die Reaktionsgeschwindigkeit der erfindungsgemäßen Formulierungen erhöht wird. Besonders bevorzugt ist hierbei der Zusatz von Trimethylolpropanoxetan (TMPO), welches auch unter dem Namen 3-Ethyl-3-oxetanmethanol bekannt ist.

Für die photochemisch induzierte Härtung der erfindungsgemäßen Formulierungen wird der Lichtwellenlängenbereich mit dem die Härtung induziert wird durch die Struktur des Initiators bestimmt. Hier ist meist die Struktur des Kations entscheidend und der Absorptionsbereich, als notwendige Voraussetzung für die photochemische Aktivität, lässt sich durch Strukturvariation des Kations in hohem Maße einstellen. Dies ist aber gegebenenfalls nachteilig, da hierdurch auch andere Eigenschaften, wie z.B. die Farbe oder die Aktivierungsgeschwindigkeit beeinflusst werden. Durch den Einsatz von Sensibilisatoren in den erfindungsgemäßen Formulierungen kann der Wellenlängenbereich in dem die photochemische Initiierung erfolgt an das Spektrum der zur Verfügung stehenden Lichtquelle angepasst werden. Die Sensibilisatoren werden in Mengen von 0,01 bis 5 Gew.-% zugesetzt, bevorzugt in Mengen von 0,1 bis 2 Gew.-%. Bevorzugte Sensibilisatoren sind Antracenderivate, besonders bevorzugt Dibutoxyanthracen und 2-Ethyldiethoxyanthracen, Naphthalimide, Benzophenone, Thioxanthone, Thiopyriliumsalze, Violanthrone, Benzoinether, Dihydropyrene, besonders bevorzugt 2,7-Di-tert-butyldimethyldihydropyren, Phenazine, Perylene, Campherchinon, Bordipyromethene, Boranile und Phthalimide, besonders bevorzugt 3-Amino-4-cyano-5-(4-methyoxyphenyl)phthalimid.

Zur Erhöhung der Aktivität der Initiatoren können den erfindungsgemäßen Formulierungen redoxaktive Substanzen zugesetzt werden. Dies kann sowohl für die thermische als auch photochemische Initiierung genutzt werden. Antioxidantien wie Irganox® 1010 oder Ascorbinsäure und deren Derivate, besonders bevorzugt Ascorbinsäurehexadecanat, sind hier ebenso bevorzugte Komponenten wie organische Kupfersalze, besonders bevorzugt Kupfer-II-benzoat, und Hydrosilane. Letztere sind Substanzen, bei denen eine oder mehrere Si-H-Bindungen vorliegen. Besonders bevorzugte Hydrosilane sind Silikone, welche SiH(CH₃)O oder SiHPhO Struktureinheiten aufweisen, Tetramethylcyclotetrasiloxan, Trimethylcyclotrisiloxan, Pentamethycyclopentasiloxan, Tetraphenylcyclotetrasiloxan, Triphenylcyclotrisiloxan und Tetramethyldisiloxan. Die redoxaktiven Substanzen werden bevorzugt in einem Anteil von 0,01 bis 5 Gew.-%, weiter vorzugsweise 0,1 bis 2 Gew.-% bezogen auf die Formulierung zugegeben.

Des Weiteren können die im Stand der Technik bei Reaktivformulierungen üblichen Formulierungsbestandteile enthalten sein.

Hier seien genannt Füllstoffe; beispielweise Kreide, Quarzpulver, Ruß, Silberpulver, pyrogene Kieselsäure; Polymere, beispielweise Gummiderivate, Polyethylenpulver, Silikone, Polyurethane; Farbstoffe oder Farbpigmente und Additive, beispielweise Verlaufsmittel, UV-Stabilisatoren oder Haftvermittler.

Die erfindungsgemäßen Formulierungen werden durch Mischen der Komponenten in den dafür üblichen Geräten hergestellt. Die Auswahl der Mischtechnik hängt im Wesentlichen von der Viskosität der Formulierung ab, welche wiederum von der Zielanwendung bestimmt ist. Bei niederviskosen Mischungen, welche insbesondere als Beschichtungsmaterialien, Vergussmassen oder Underfiller für die Elektronik einsetzbar sind, erfolgt die Durchmischung der Komponenten mit einem einfachen Rührer oder Dissolver. Wenn Füllstoffe fein verteilt werden sollen oder auch wenn der Initiator in der Reaktivmischung schwer löslich ist, kann der Einsatz einer Kugelmühle die beste Wahl sein. Bei Klebstoffpasten ist die Fertigung der Formulierung in einem Kneter zielführend.

Die Applikation der Formulierungen ist anwendungsspezifisch und die Applikationstechnik hängt eng mit der Viskosität zusammen. Bei Elektronikanwendungen sind Dispensen, die Applikation mittels Inkjet oder der Tampondruck passende Applikationstechniken. Bei größeren benötigten Mengen, z.B. der Applikation von Klebraupen im Automobilbau, werden die einschlägigen im Stand der Technik bekannten Dosieranlagen verwendet. Die schichtförmige Applikation, wie man sie bei der Herstellung von Laminaten oder Druckprodukten benötigt, kann durch Sprühapplikation, Rakeln oder Druckwalzen erfolgen. Des Weiteren sind alle im Stand der Technik bekannten Applikationsverfahren geeignet, um die erfindungsgemäßen Formulierungen zu verarbeiten, angepasst auf die Anwendung und die Viskosität sowie zu applizierende Menge der Formulierungen.

Nach der Applikation erfolgt die Härtung der erfindungsgemäßen Formulierungen. Hierfür werden die enthaltenen Initiatoren entweder thermisch oder photochemisch aktiviert und starten die Härtungsreaktion. Die thermische Härtung erfolgt typischerweise in einem Ofen. Die passende Härtungstemperatur und notwendige Zeit wird entweder empirisch ermittelt oder anhand von DSC Daten, in für den Fachmann in an sich bekannter Weise, bestimmt. Hierfür wird zunächst eine dynamische DSC Messung der Formulierung durchgeführt. Es werden der Start und das Maximum des Reaktionspeaks in der DSC ermittelt. Wenn eine minimale Ofentemperatur gewünscht ist, kann eine Temperatur von etwa 10°C unterhalb des Startes erprobt werden. Dies kann bei empfindlichen Bauteilen oder großen Mengen der Formulierung notwendig sein, da in dem Fall eine Eigenerwärmung durch die Reaktionsenthalpie zu erwarten ist. In diesem Fall werden relativ lange Zeiten zur Härtung benötigt. Wenn das Härtungsergebnis nicht zufriedenstellend ist, muss die Temperatur in Schritten erhöht werden, bis ein zufriedenstellendes Härtungsergebnis bei vorgegebener Zeit vorliegt. Ob die Härtung vollständig ist wird wiederum mittels DSC ermittelt. Wenn eine vollständige Härtung gewünscht ist, sollte keine Restreaktionsenthalpie bei der gehärteten Formulierung mehr auftreten. Gegebenenfalls muss mit einem Wärmeprofil für eine vollständige Härtung gearbeitet werden, dies ist immer dann ratsam wenn z.B. aufgrund einer großen Menge die Härtungsreaktion aufgrund der Eigenerwärmung zu einer ungewünschten Temperaturspitze führt. In dem Fall wird die Temperatur schrittweise gesteigert. Bei kleinen zu härtenden Mengen der Formulierung ist die mit DSC bestimmte Peaktemperatur ein guter Startwert für die ansonsten empirisch zu ermittelnde Ofentemperatur.

Die photochemische Härtung erfolgt mit Lichtquellen im sichtbaren oder UV-Bereich des elektromagnetischen Spektrums. Es kann sich um polychromatische Lichtquellen wie Quecksilberdampflampen oder auch monochromatische Lichtquellen wie LED-Strahler, Laser oder Eximerlampen handeln. Der für die Formulierung auszuwählende Initiator oder auch die Kombination aus Initiator und Sensibilisator muss in dem Emissionsbereich der Strahlenquelle absorbieren. Die Photohärtung ist besonders bei dünnen aufgetragenen Schichten der Formulierung vorteilhaft und erfolgt durch Bestahlen mit der ausgewählten Lampe. Die Härtung erfolgt umso schneller, je höher die Lichtintensität gewählt wird. Hierbei kommt es üblicherweise auch zu einer Erwärmung des Substrates, was die Härtung unterstützt, bei einer zu großen Intensität aber auch die Substrate oder gehärtete Formulierung schädigen kann.

Es kann auch eine sogenannte Dualhärtung durchgeführt werden. Hierbei wird zunächst photochemisch an durch Licht erreichbaren Stellen vorgehärtet, um eine Handlingfestigkeit zu erzielen. Die Durchhärtung und Härtung an den abgeschatteten Bereichen erfolgt anschließend thermisch. Die erfindungsgemäßen Initiatoren und damit hergestellten Formulierungen haben den Vorteil, dass sie sowohl photochemisch als auch thermisch gehärtet werden können.

Ein weiterer Gegenstand der Erfindung ist die Verwendung einer erfindungsgemäßen Formulierung als Klebstoffe, Beschichtungsmaterialien, Matrixharze für Verbundwerkstoffe, oder Vergussmassen; oder bei der Herstellung von Klebstoffen, Beschichtungsmaterialien, Matrixharzen für Verbundwerkstoffe, oder Vergussmassen

Der Anwendungsbereich der erfindungsgemäßen Formulierungen ist sehr vielfältig. Bei Lebensmittelverpackungen sind die Abwesenheit von Schwermetallen und hydrolysierbarem Fluorid ein Vorteil. Hier kann der Einsatz bei photohärtenden Druckfarben und Kaschierklebstoffen erfolgen. Im Bereich von Klebstoffen, Laminierharzen, Vergussmassen für die allgemeine Industrie sind dies neben der hohen Latenz ebenso große Vorteile. In der Elektrotechnik und Elektronik, wo die erfindungsgemäßen Formulierungen als Klebstoffe, Vergussmassen, Beschichtungsmaterialien, Verkapselungen oder Underfiller genutzt werden können, kann es durch hydrolysierbares Fluorid aus nach dem Stand der Technik bekannten Initiatoren zu Korrosion kommen und auch Schwermetalle wie Antimon sind zunehmend nicht gewünscht. Diese Nachteile können durch die erfindungsgemäßen Formulierungen überwunden werden.

Die Erfindung wird nachfolgend anhand von Ausführungsbeispielen erläutert.

### Beispiel 1

### Synthese von Li Al(OC(CF₃)₃)₄ als Ausgangsstoff nach dem Stand der Technik

Die Reaktion wurde in einem 100 ml Dreihalskolben unter N₂ Atmosphäre durchgeführt. LiAlH₄ (0,53 g, 14mmol) wurde in Toluol (70 ml) suspendiert und der Alkohol HOC(CF₃)₃ (13,52g, 8,0 ml, 57,3 mmol) wurde bei 0°C (Eis + Wasser 1:1) unter Rühren mit einem Magnetrührer zugetropft. Hierbei ist Gasentwicklung zu beobachten. Die Mischung wurde dem Eisbad entnommen, auf Raumtemperatur gebracht und dann 7 Stunden unter Rückfluss erhitzt. Es wurde einmal mit 50 ml Wasser ausgeschüttelt und dann das Lösemittel von der organischen Phase am Rotationsverdampfer abgezogen. Es bleibt ein weißes Pulver als Rückstand zurück, welches für die Synthese der Initiatoren eingesetzt wird.

### Beispiel 2

### Synthese von Lithium tetrakis(pentafluorophenoxo)aluminat als Ausgangsstoff nach dem Stand der Technik

12,7 g (334 mmol) Lithiumaluminiumchlorid werden in 600 ml Toluol suspendiert und eine Lösung von 16 g Pentafluorphenol (84 mmol) gelöst in 100 ml Toluol werden unter Rühren mit einem Magnetrührer so zugetropft, dass die Temperatur 50°C nicht übersteigt. Hierbei ist eine Gasentwicklung zu beobachten. Anschließend wird 10 Stunden bei einer Ölbadtemperatur von 95°C gerührt. Nach dem Abkühlen wird einmal mit 200 ml Wasser ausgeschüttelt und von der organischen Phase das Lösemittel am Rotationsverdampfer abgezogen. Das resultierende fast weiße Pulver wird für die Synthese der Initiatoren durch Metathese eingesetzt.

### Beispiel 3

### Synthese von Bis-(4-methylphenyl)iodonium) [Al(O-C(CF₃)₃)₄] durch Metathese in einem organischen Lösemittel und Photohärtung von Epoxidharzen

0,405 g (1 mmol) des frisch hergestellten Bis-(4-methylphenyl)iodonium) hydrogensulfates wird in der 3 ml Acetonitril suspendiert. 1,12 g (1,15 mmol) des Li[Al(OC(CF₃)₃)₄] werden unter Rühren zu dieser Suspension gegeben und 5 Stunden bei Raumtemperatur mit einem Magnetrührer gerührt. Es wird eine gelbliche Lösung erhalten, von der die gebildeten Feststoffe abfiltriert werden. Die Reaktionslösung wird eingedampft. Das erhaltene Produkt wird in Dichlormethan aufgenommen und mit Wasser gewaschen. 1,2 g des Produktes werden nach dem Eindampfen des Dichlormethans erhalten.

Es wird eine 1%ige klare Lösung des Produktes in 3,4-Epoxycyclohexyl-3',4'-epoxycyclohexane carboxylat präpariert. Ebenso wird eine 1%ige Lösung des als Initiator wirkenden Produktes in epoxidiertem Leinöl präpariert. Auch hier entsteht eine klare Lösung. Ein besonderer Vorteil dieses Initiators ist die sehr gute Löslichkeit in den kationisch härtbaren Bindemitteln.

Die Formulierungen werden mit einem Rakel in einer Schichtdicke von 100 µm auf Glasscheiben appliziert und 30 s mit einem UV-Strahler OmniCure Series 2000 in einem Abstand von 5 cm bestrahlt. Es resultieren harte gut haftende Schichten auf dem Glas.

### Beispiel 4

### Synthese von Bis-(4-isobutylphenyl)iodonium [Al(O-C(CF₃)₃)₄] durch Metathese in einem organischen Lösemittel und Photohärtung von einem Epoxidharz und einem Vinylether

0,452 g (1 mmol) des frisch hergestellten Bis-(4-isobutylphenyl)iodonium) acetats werden in 2,5 ml Acetonitril suspendiert. 1,12 g (1,15 mmol) des Li[Al(OC(CF₃)₃)₄] werden unter Rühren zu dieser Suspension gegeben und 5 Stunden bei Raumtemperatur mit einem Magnetrührer gerührt. Es wird eine gelbliche Lösung erhalten, von der die gebildeten Feststoffe abfiltriert werden. Die Reaktionslösung wird eingedampft. Das erhaltene Produkt wird in Dichlormethan aufgenommen und mit Wasser gewaschen. 1,35 g des Produktes werden nach dem Eindampfen des Dichlormethans erhalten und als Initiator für photohärtbare Formulierungen verwendet.

Es wird eine 1%ige klare Lösung des Produktes in 3,4-Epoxycyclohexyl-3',4'-epoxycyclohexane carboxylat präpariert. Ebenso wird eine 1%ige Lösung des Initiators in Triethylenglycoldivinylether präpariert. Auch hier entsteht eine klare Lösung. Ein besonderer Vorteil dieses Initiators ist die sehr gute Löslichkeit in den kationisch härtbaren Bindemitteln.

Die Formulierung basierend auf 3,4-Epoxycyclohexyl-3',4'-epoxycyclohexane carboxylat wird mit einem Rakel in einer Schichtdicke von 100 µm auf eine Glasscheibe appliziert und die Formulierung basierend auf dem Vinylether in einer Aluminiumschale in dünner Schicht verteilt. Beide applizierten Formulierungen werden für 30 s mit einem UV-Strahler OmniCure Series 2000 in einem Abstand von 5 cm bestrahlt. Bei einem zweiten Ansatz wird mit einem LED Strahler Cyberbond Linop U400 für 30 s und 5 cm Abstand bestrahlt. In allen Fällen sind die applizierten Schichten ausgehärtet.

### Beispiel 5

### Synthese von (9-Oxo-9H-fluoren-2-yl) phenyl iodonium tetrakis(pentafluorophenoxo) aluminat durch Metathese in einem organischen Lösemittel und Photohärtung von einem Epoxidharz

10 g (23 mmol) (9-Oxo-9H-fluoren-2-yl) phenyl iodonium chlorid werden nach dem Stand der Technik präpariert (A. Hartwig et al. Eur. Polym. J. 37 (2001) 1449) und in 50 ml Aceton dispergiert und 17,62 g (23 mmol) Lithium tetrakis(pentafluorophenoxo) aluminat hinzu gegeben. Die Aufschlämmung wird 10 Stunden bei Raumtemperatur gerührt und dann 200 ml Dichlormethan zugegeben. Anschließend wird zweimal mit je 70 ml Wasser ausgeschüttelt und das Lösemittel von der organischen Phase abgezogen. Das resultierende kanariengelbe Pulver wird ohne weitere Aufarbeitung als Photoinitiator eingesetzt.

Es wird eine 1%ige Lösung des Initiators in einer Mischung aus 80 Gewichtsteilen 3,4-Epoxycyclohexyl-3',4'-epoxycyclohexane carboxylat und 20 Gewichtsteilen Polycaprolactondiol mit der Molmasse 400 präpariert. Diese Formulierung wird als 100 µm dicke Schicht auf einer Glasscheibe ausgerakelt und mit einem LED Strahler Cyberbond Linop U400 für 30 s bei 5 cm Abstand bestrahlt. Es resultiert eine zähharte Schicht auf dem Glas.

### Beispiel 6

### Synthese von p-Methoxybenzyl tetrahydrothiophenium [Al(O-C(CF₃)₃)₄] durch Metathese in Wasser und Präparation von Formulierungen

5,13 mmol (5 g) Li [Al(O-C(CF₃)₃)₄] wurden in 100 ml dest. Wasser gelöst und langsam unter Rühren mit einem Magnetrührer eine Lösung von 5,15 mmol (1,26 g) p-Methoxybenzyl tetrahydrothiophenium chlorid in 100 ml Wasser gegeben. Es fällt ein weißer Niederschlag aus, der abfiltriert und mit destilliertem Wasser gewaschen wird bis das Waschwasser chloridfrei ist (Silbernitrattest). Der Niederschlag wurde bei 40°C im Umluftofen bis zur Massenkonstanz getrocknet (8 Stunden). Es werden 4,25 g Initiator erhalten.

Es wurde eine 1 %ige Lösung des Initiators in 3,4-Epoxycyclohexyl-3',4'-epoxycyclohexane carboxylat präpariert. Hierbei entsteht eine klare Lösung. Mittels DSC (10 K/min) wird ein Onset der Härtungsreaktion bei 72°C und ein Peakmaximum bei 80°C festgestellt.

0,5 g der Formulierung wurden in eine Aluminiumschale gegeben und für 30 min im Ofen bei 100°C gehärtet. Es resultiert ein hartes leicht bräunliches Polymer.

### Beispiel 6a - Vergleichsbeispiel

1 Gew.-% p-Methoxybenzyl tetrahydrothiophenium SbF₆ nach dem Stand der Technik wurde in 3,4-Epoxycyclohexyl-3',4'-epoxycyclohexane carboxylat gelöst. Mittels DSC (10 K/min) wird ein Onset der Härtungsreaktion bei 75°C und ein Peakmaximum bei 93°C festgestellt. 0,5 g der Formulierung wurden in eine Aluminiumschale gegeben und für 30 min im Ofen bei 100°C gehärtet. Es resultiert ein hartes Polymer.

Der Vergleichsversuch zeigt, dass die erfindungsgemäße Formulierung mit dem neuen Initiator zu gleichen Härtungseigenschaften führt wie der Initiator mit dem nachteiligen Anion SbF₆⁻.

### Beispiel 7

### Synthese von Benzyl tetrahydrothiophenium [Al(O-C(CF₃)₃)₄] durch Metathese in Wasser und Präparation von Formulierungen

5,13 mmol (5 g) Li [Al(O-C(CF₃)₃)₄] wurden in 100 ml dest. Wasser gelöst und langsam unter Rühren mit einem Magnetrührer eine Lösung von 5,13 mmol (1,33 g) Benzyl tetrahydrothiophenium bromid in 100 ml Wasser gegeben. Es fällt ein weißer Niederschlag aus, der abfiltriert und mit destilliertem Wasser gewaschen wird, bis das Waschwasser bromidfrei ist (Silbernitrattest). Der Niederschlag wurde bei 40°C im Umluftofen bis zur Massenkonstanz getrocknet (8 Stunden). Es werden 3,2 g Initiator erhalten.

Es wurde eine 1 %ige Lösung des Initiators in 3,4-Epoxycyclohexyl-3',4'-epoxycyclohexane carboxylat präpariert. Hierbei entsteht eine klare Lösung. Mittels DSC (10 K/min) wird ein Onset der Härtungsreaktion bei 132°C und ein Peakmaximum bei 156°C festgestellt.

0,5 g der Formulierung wurden in eine Aluminiumschale gegeben und für 30 min im Ofen bei 140°C gehärtet. Es resultiert ein hartes leicht bräunliches Polymer.

Es wurde eine Formulierung präpariert, die 1 Gew.-% des Initiators Benzyl tetrahydrothiophenium [Al(O-C(CF₃)₃)₄], 1 Gew.-% Ascorbinsäurehexadecanat als Beschleuniger und 98 Gew.-% 3,4-Epoxycyclohexyl-3',4'-epoxycyclohexane carboxylat beinhaltet. Hierbei entsteht eine klare Lösung. Mittels DSC (10 K/min) wird ein Onset der Härtungsreaktion bei 127°C und ein Peakmaximum bei 144°C festgestellt. Gegenüber der nicht mit dem Ascorbinsäurehexadecanat modifizierten Formulierung ergibt sich demnach eine niedrigere Härtungstemperatur, das Ascorbinsäurehexadecant wirkt demnach als Beschleuniger.

0,5 g der Formulierung wurden in eine Aluminiumschale gegeben und für 30 min im Ofen bei 140°C gehärtet. Es resultiert ein hartes leicht bräunliches Polymer.

### Beispiel 8

### Synthese von p-Methoxybenzyl-N,N-dimethylanilinium [Al(O-C(CF₃)₃)₄] durch Metathese in Wasser und Präparation von Formulierungen

5,13 mmol (5 g) Li [Al(O-C(CF₃)₃)₄] wurden in 100 ml dest. Wasser gelöst und langsam unter Rühren mit einem Magnetrührer eine Lösung von 5,15 mmol (1,43 g) p-Methoxybenzyl-N,N-dimethylanilinium chlorid in 100 ml Wasser gegeben. Es fällt ein weißer Niederschlag aus, der abfiltriert und mit destilliertem Wasser gewaschen wird, bis das Waschwasser chloridfrei ist (Silbernitrattest). Der Niederschlag wurde bei 40°C im Umluftofen bis zur Massenkonstanz getrocknet (8 Stunden). Es werden 4,39 g Initiator erhalten.

Es wurde eine 1 %ige Lösung des Initiators in 3,4-Epoxycyclohexyl-3',4'-epoxycyclohexane carboxylat präpariert. Hierbei entsteht eine klare Lösung. Mittels DSC (10 K/min) wird ein Onset der Härtungsreaktion bei 107°C und ein Peakmaximum bei 113°C festgestellt.

0,5 g der Formulierung wurden in eine Aluminiumschale gegeben und für 30 min im Ofen bei 100°C gehärtet. Es resultiert ein hartes leicht gelbes Polymer.

Es wurde eine Formulierung bereitet, die 1 % Gew.-% des Initiators, 79 Gew.-% 3,4-Epoxycyclohexyl-3',4'-epoxycyclohexane carboxylat und 20 Gew.-% Polycaprolactondiol mit der mittleren Molmasse 400 (PCL 400 von Perstorp) enthielt. Es entsteht eine transparente farblose Formulierung. Mittels DSC (10 K/min) wird ein Onset der Härtungsreaktion bei 107°C und ein Peakmaximum bei 115°C festgestellt.

0,5 g der Formulierung wurden in eine Aluminiumschale gegeben und für 30 min im Ofen bei 100°C gehärtet. Es resultiert ein zähhartes Polymer welches eine hohe Haftung zu dem Aluminium aufweist.

## Patentansprüche

1. Formulierung, **dadurch gekennzeichnet, dass** sie kationisch polymerisierbare Monomere, Oligomere und/oder Polymere, eine Zusammensetzung, enthaltend
a. ein Anion der Formel
[E(ZR1)ₙ(ZR2)ₘ]
mit:
E gleich Al oder B,
Z gleich 0 oder S,
R1, R2 gleiche oder verschiedene organische Reste mit:
- R1 1 bis 30 C-Atomen und R2 2 bis 30 C-Atomen,
- Bestandteilen ausgewählt aus der Gruppe bestehend aus gesättigten und ungesättigten, unverzweigten und verzweigten Aliphaten, Alicyclen, Heterocyclen und Aromaten,
- optional versehen mit Homo- und/oder Heterosubstituenten,
wobei in R2 wenigstens vier der zur Absättigung der C-Atome erforderlichen H durch F ersetzt sind;
m gleich oder größer 2,
m + n = 4,
b. ein Kation einer der Formeln:
(Rx)₂I⁺, (Rx)₃S⁺, (Rx)₄P⁺, oder (Rx)₄N⁺
mit Rx ausgewählt aus der Gruppe bestehend aus R4, R5, R6 und R7,
wobei R4, R5, R6 und R7 gleiche oder verschiedene organische Reste sind, mit:
- 1 bis 30 C-Atomen,
- Bestandteilen ausgewählt aus der Gruppe bestehend aus gesättigten und ungesättigten, unverzweigten und verzweigten Aliphaten, Alicyclen, Heterocyclen und Aromaten,
- optional versehen mit Homo- und/oder Heterosubstituenten,
wobei wenigstens ein Rx einen aromatischen Ring aufweist,
sowie Polyole in einem Anteil von 5 bis 80 Gew.-% der Formulierung enthält, wobei bevorzugt die kationisch polymerisierbaren Monomere, Oligomere und/oder Präpolymere ausgewählt sind aus der Gruppe bestehend aus Epoxidharzen und Vinylethern.

2. Formulierung nach Anspruch 1, **gekennzeichnet durch** eines oder mehrere der folgenden Merkmale:
a. R2 weist 3 bis 15 C-Atome auf,
b. in R2 sind sind wenigstens die Hälfte, vorzugsweise alle der zur Absättigung der C-Atome erforderlichen H durch F ersetzt,
c. m=4,
d. R2 ist ausgewählt aus der Gruppe bestehend aus -C(CF₃)₃, -CH(CF₃)₂, -C(CH₃)(CF₃)₂, -CPh(CF₃)₂, -CPh₂(CF₃), -CPhCH₃(CF₃)₂, -CPhCF₃(CF₃)₂, -PhF₅, -PhF₄, und -PhClF₄,
e. R2 ist ausgewählt aus der Gruppe bestehend aus 4-Methyltetrafluorbenzoxy, 4-Trifluormethylbenzoxy, 2,3,5,6-Tetrafluorbenzoxy, und 4-Chlortetrafluorbenzoxy.

3. Formulierung nach Anspruch 1 oder 2, **dadurch gekennzeichnet, dass** je zwei R1 und/oder R2 einen bidentalen Rest R3 bilden, der zwei Bindungsstellen über jeweils ein Sauerstoffatom zu E aufweist.

4. Formulierung nach einem der Ansprüche 1 bis 3, **dadurch gekennzeichnet, dass** Rx 3 bis 15 C-Atome aufweist und/oder dass zwei Rx einen bidentalen Rest bilden.

5. Formulierung nach einem der Ansprüche 1 bis 4, **dadurch gekennzeichnet, dass** sie die Zusammensetzung nach einem der Ansprüche 1 bis 4 in einem Anteil von 0,05 bis 10 Gew.-%, vorzugsweise 0,2 bis 5 Gew.-%, enthält.

6. Formulierung nach einem der Ansprüche 1 bis 5, **dadurch gekennzeichnet, dass** die Epoxidharze epoxidierte Pflanzenöle oder cycloaliphatische Epoxidharze sind, bevorzugt ausgewählt aus der Gruppe bestehend aus 3,4-Epoxycyclohexyl-3',4'-epoxycyclohexancarboxylat und Bis-(3,4-epoxycyclohexylmethyl)adipat.

7. Formulierung nach einem der Ansprüche 1 bis 5, **dadurch gekennzeichnet, dass** die Vinylether ausgewählt sind aus der Gruppe bestehend aus Cyclohexylvinylether, Butandiolvinylether, und Triethylenglycoldivinylether.

8. Formulierung nach einem der Ansprüche 1 bis 7, **dadurch gekennzeichnet, dass** sie Polyolein einem Anteil von 10 bis 50 Gew.-% der Formulierung enthält; wobei die Polyole vorzugsweise 2 bis 5 OH-Gruppen, weiter vorzugsweise 2 bis 3 OH-Gruppen je Molekül aufweisen.

9. Formulierung nach einem der Ansprüche 1 bis 8, **dadurch gekennzeichnet, dass** sie zusätzlich Reduktionsmittel enthält; bevorzugt ausgewählt aus der Gruppe bestehend aus Ascorbinsäure und deren Derivaten, organischen Kupfersalzen, und Hydrosilanen; bevorzugt in einem Anteil von 0,01 bis 5 Gew.-%, weiter vorzugsweise 0,1 bis 2 Gew.-%.

10. Formulierung nach einem der Ansprüche 1 bis 9, **dadurch gekennzeichnet, dass** sie zusätzlich Photosensibilatoren enthält; bevorzugt in einem Anteil von 0,01 bis 5 Gew.-%, weiter vorzugsweise 0,1 bis 2 Gew.-%; wobei die Photosensibilatoren bevorzugt ausgewählt sind aus der Gruppe bestehend aus Antracenderivaten, Naphthalimiden, Benzophenonen, Thioxanthonen, Thiopyriliumsalzen, Violanthronen, Benzoinether, Dihydropyrenen, Phenazinen, Perylenen, Campherchinon, Bordipyromethenen, Boranilen und Phthalimiden.

11. Verwendung einer Formulierung nach einem der Ansprüche 1 bis 10 als Klebstoffe, Beschichtungsmaterialien, Matrixharze für Verbundwerkstoffe, oder Vergussmassen; oder bei der Herstellung von Klebstoffen, Beschichtungsmaterialien, Matrixharzen für Verbundwerkstoffe, oder Vergussmassen.

## Claims

1. Formulation, **characterized in that** it comprises cationically polymerizable monomers, oligomers and/or polymers, a composition comprising
a. an anion of the formula
[E(ZR1)ₙ(ZR2)ₘ]
where:
E is Al or B,
Z is 0 or S,
R1, R2 are identical or different organic radicals with:
- R1 from 1 to 30 carbon atoms and R2 from 2 to 30 carbon atoms,
- constituents selected from the group consisting of saturated and unsaturated, unbranched and branched aliphatics, alicycles, heterocycles and aromatics,
- optionally provided with homo- and/or hetero-substituents,
wherein in R2 at least four of the H atoms required for saturation of the C atoms have been replaced by F;
m is equal to or greater than 2,
m + n = 4,
b. a cation of one of the formulae:
(Rx)₂I⁺, (Rx)₃S⁺, (Rx)₄P⁺ or (Rx)₄N⁺
with Rx selected from the group consisting of R4, R5, R6 and R7,
wherein R4, R5, R6 and R7 are identical or different organic radicals with:
- from 1 to 30 carbon atoms,
- constituents selected from the group consisting of saturated and unsaturated, unbranched and branched aliphatics, alicycles, heterocycles and aromatics,
- optionally provided with homo- and/or hetero-substituents,
wherein at least one Rx has an aromatic ring,
as well as polyols in an amount of from 5 to 80% by weight of the formulation, wherein preferably the cationically polymerizable monomers, oligomers and/or prepolymers are selected from the group consisting of epoxy resins and vinyl ethers.

2. Formulation according to Claim 1, **characterized by** one or more of the following features:
a. R2 has from 3 to 15 carbon atoms,
b. in R2, at least half, preferably all, of the H atoms required for saturation of the C atoms have been have been replaced by F,
c. m = 4,
d. R2 is selected from the group consisting of -C(CF₃)₃, -CH(CF₃)₂, -C(CH₃)(CF₃)₂,-CPh(CF₃)₂, -CPh₂(CF₃), -CPhCH₃ (CF₃)₂, -CPhCF₃(CF₃)₂,-PhF₅, -PhF₄ and -PhClF₄,
e. R2 is selected from the group consisting of 4-methyltetrafluorobenzoxy, 4-trifluoromethylbenzoxy, 2,3,5,6-tetrafluorobenzoxy and 4-chlorotetrafluorobenzoxy.

3. Formulation according to Claim 1 or 2, **characterized in that** in each case two R1 and/or R2 form a bidentate radical R3 which has two binding sites to E in each case *via* an oxygen atom.

4. Formulation according to one of Claims 1 to 3, **characterized in that** Rx contains from 3 to 15 carbon atoms and/or **in that** two Rx form a bidentate radical.

5. Formulation according to one of Claims 1 to 4, **characterized in that** it comprises the composition according to one of Claims 1 to 4 in an amount of from 0.05 to 10% by weight, preferably from 0.2 to 5% by weight.

6. Formulation according to one of Claims 1 to 5, **characterized in that** the epoxy resins are epoxidized vegetable oils or cycloaliphatic epoxy resins, preferably selected from the group consisting of 3,4-epoxycyclohexyl-3',4'-epoxycyclohexane carboxylate and bis-(3,4-epoxycyclohexylmethyl) adipate.

7. Formulation according to one of Claims 1 to 5, **characterized in that** the vinyl ethers are selected from the group consisting of cyclohexyl vinyl ether, butanediol vinyl ether and triethylene glycol divinyl ether.

8. Formulation according to one of Claims 1 to 7, **characterized in that** it comprises polyols in an amount of from 10 to 50% by weight of the formulation; wherein the polyols contain preferably from 2 to 5 OH groups, further preferably from 2 to 3 OH groups, per molecule.

9. Formulation according to one of Claims 1 to 8, **characterized in that** it additionally comprises reducing agents; preferably selected from the group consisting of ascorbic acid and derivatives thereof, organic copper salts and hydrosilanes; preferably in an amount of from 0.01 to 5% by weight, further preferably from 0.1 to 2% by weight.

10. Formulation according to one of Claims 1 to 9, **characterized in that** it additionally comprises photosensitizers; preferably in an amount of from 0.01 to 5% by weight, further preferably from 0.1 to 2% by weight; wherein the photosensitizers are preferably selected from the group consisting of anthracene derivatives, naphthalimides, benzophenones, thioxanthones, thiopyrylium salts, violanthrones, benzoin ethers, dihydropyrenes, phenazines, perylenes, camphorquinone, boron dipyromethenes, boranils and phthalimides.

11. Use of a formulation according to one of Claims 1 to 10 as adhesives, coating materials, matrix resins for composite materials, or sealing compounds; or in the production of adhesives, coating materials, matrix resins for composite materials, or sealing compounds.

## Revendications

1. Formulation, **caractérisée en ce qu'**elle contient des monomères polymérisables cationiquement, des oligomères et/ou des polymères, une composition, contenant :
a. un anion de formule
[E(ZR1)ₙ(ZR2)ₘ]
avec :
E = Al ou B,
Z = 0 ou S,
R1, R2 = des radicaux organiques identiques ou différents, contenant :
- R1 1 à 30 atomes C et R2 2 à 30 atomes C,
- des constituants choisis dans le groupe constitué par les groupes aliphatiques saturés et insaturés, non ramifiés et ramifiés, les groupes alicycliques, les groupes hétérocycliques et les groupes aromatiques,
- éventuellement munis d'homo- et/ou d'hétéro-substituants,
dans R2, au moins quatre des H nécessaires pour la saturation des atomes C étant remplacés par des F ;
m supérieur ou égal à 2,
m + n = 4,
b. un cation d'une des formules :
(Rx)₂I⁺, (Rx)₃S⁺, (Rx)₄P⁺ ou (Rx)₄N⁺
avec Rx choisi dans le groupe constitué par R4, R5, R6 et R7,
R4, R5, R6 et R7 étant des radicaux organiques identiques ou différents, contenant :
- 1 à 30 atomes C,
- des constituants choisis dans le groupe constitué par les groupes aliphatiques saturés et insaturés, non ramifiés et ramifiés, les groupes alicycliques, les groupes hétérocycliques et les groupes aromatiques,
- éventuellement munis d'homo- et/ou d'hétéro-substituants,
au moins un Rx comprenant un cycle aromatique,
ainsi que des polyols en une proportion de 5 à 80 % en poids de la formulation ; les monomères polymérisables cationiquement, les oligomères et/ou les prépolymères étant de préférence choisis dans le groupe constitué par les résines époxydes et les éthers de vinyle.

2. Formulation selon la revendication 1, **caractérisée par** une ou plusieurs des caractéristiques suivantes :
a. R2 comprend 3 à 15 atomes C,
b. dans R2, au moins la moitié, de préférence tous les H nécessaires pour la saturation des atomes C sont sont remplacés par des F,
c. m = 4,
d. R2 est choisi dans le groupe constitué par-C(CF₃)₃, -CH(CF₃)₂, -C(CH₃)(CF₃)₂, -CPh(CF₃)₂, -CPh₂(CF₃), -CPhCH₃ (CF₃)₂, -CPhCF₃(CF₃)₂, -PhF₅, -PhF₄ et -PhClF₄,
e. R2 est choisi dans le groupe constitué par le 4-méthyltétrafluorobenzoxy, le 4-trifluorométhylbenzoxy, le 2,3,5,6-tétrafluorobenzoxy et le 4-chlorotétrafluorobenzoxy.

3. Formulation selon la revendication 1 ou 2, **caractérisée en ce que** deux R1 et/ou R2 forment respectivement un radical bidentate R3, qui comprend deux emplacements de liaison à E à chaque fois par le biais d'un atome d'oxygène.

4. Formulation selon l'une quelconque des revendications 1 à 3, **caractérisée en ce que** Rx comprend 3 à 15 atomes C et/ou **en ce que** deux Rx forment un radical bidentate.

5. Formulation selon l'une quelconque des revendications 1 à 4, **caractérisée en ce qu'**elle contient la composition selon l'une quelconque des revendications 1 à 4 en une proportion de 0,05 à 10 % en poids, de préférence de 0,2 à 5 % en poids.

6. Formulation selon l'une quelconque des revendications 1 à 5, **caractérisée en ce que** les résines époxydes sont des huiles végétales époxydées ou des résines époxydes cycloaliphatiques, de préférence choisies dans le groupe constitué par le carboxylate de 3,4-époxycyclohexyl-3',4'-époxycyclohexane et l'adipate de bis(3,4-époxycyclohexylméthyle).

7. Formulation selon l'une quelconque des revendications 1 à 5, **caractérisée en ce que** les éthers de vinyle sont choisis dans le groupe constitué par l'éther vinylique de cyclohexyle, l'éther vinylique de butanediol et l'éther divinylique de triéthylène glycol.

8. Formulation selon l'une quelconque des revendications 1 à 7, **caractérisée en ce qu'**elle contient des polyols en une proportion de 10 à 50 % en poids de la formulation ; les polyols comprenant de préférence 2 à 5 groupes OH, de manière davantage préférée 2 à 3 groupes OH, par molécule.

9. Formulation selon l'une quelconque des revendications 1 à 8, **caractérisée en ce qu'**elle contient en outre des réducteurs ; de préférence choisis dans le groupe constitué par l'acide ascorbique et ses dérivés, les sels de cuivre organiques et les hydrosilanes ; de préférence en une proportion de 0,01 à 5 % en poids, de manière davantage préférée de 0,1 à 2 % en poids.

10. Formulation selon l'une quelconque des revendications 1 à 9, **caractérisée en ce qu'**elle contient en outre des photosensibilisateurs ; de préférence en une proportion de 0,01 à 5 % en poids, de manière davantage préférée de 0,1 à 2 % en poids ; les photosensibilisateurs étant de préférence choisis dans le groupe constitué par les dérivés d'anthracène, les naphtalimides, les benzophénones, les thioxanthones, les sels de thiopyrilium, les violanthrones, les éthers de benzoïne, les dihydropyrènes, les phénazines, les pérylènes, la camphre-quinone, les borodipyrométhènes, les boranils et les phtalimides.

11. Utilisation d'une formulation selon l'une quelconque des revendications 1 à 10 en tant qu'adhésifs, matériaux de revêtement, résines de matrice pour matériaux composites ou masses de scellement ; ou lors de la fabrication d'adhésifs de matériaux de revêtement, de résines de matrice pour matériaux composites ou de masses de scellement.
